# EUROPÄISCHE PATENTSCHRIFT

(11) **EP 1 827 321 B1**
(45) Veröffentlichungstag und Bekanntmachung des Hinweises auf die Patenterteilung: **27.04.2011**
(21) Anmeldenummer: 05813865.2
(22) Anmeldetag: 26.11.2005
(51) Int. Cl.: A61F 2/44, A61F 2/30

(54) **BAUSATZ FÜR EIN ZWISCHENWIRBELIMPLANTAT UND ZWISCHENWIRBELIMPLANTAT**
KIT FOR AN INTERVERTEBRAL IMPLANT AND INTERVERTEBRAL IMPLANT
ENSEMBLE DE PIECES POUR IMPLANT INTERVERTEBRAL, ET IMPLANT INTERVERTEBRAL

(30) Priorität: 09.12.2004 DE 102004059298
(43) Veröffentlichungstag der Anmeldung: 05.09.2007
(73) Patentinhaber: Aesculap AG, 78532 Tuttlingen (DE)
(72) Erfinder: SCHNEID, Susanne, 78532 Tuttlingen (DE); SCHULTZ, Robert, 78532 Tuttlingen (DE)
(74) Vertreter: Hoeger, Stellrecht & Partner Patentanwälte
(86) Internationale Anmeldenummer: PCT/EP2005/012653
(87) Internationale Veröffentlichungsnummer: WO 2006/061114

(56) Entgegenhaltungen:
- WO-A-99/32055
- DE-U1- 20 320 454
- US-A1- 2004 133 278

## Beschreibung

Die Erfindung betrifft einen Bausatz für ein Zwischenwirbelimplantat, welches eine obere und eine untere Endplatte zur Anlage an benachbarten Wirbelkörpern sowie zwischen den Endplatten angeordnete Lagerelemente zur verschwenkbaren gegenseitigen Abstützung der Endplatten aufweist, wobei die Endplatten an ihren den Wirbelkörpern zugewandten Außenseiten Verankerungsvorsprünge tragen.

Zwischenwirbelimplantate dieser Art werden als Ersatz für geschädigte Bandscheiben zwischen benachbarte Wirbelkörper eingesetzt. Zur Verankerung der Endplatten tragen bekannte Zwischenwirbelimplantate Vorsprünge, die in die Knochensubstanz der benachbarten Wirbelkörper eingreifen, beispielsweise in Form von Spitzen oder in Form von Rippen oder Finnen, die parallel zur Einschubrichtung verlaufen (EP 1 057 462 B1; WO 02/089701 A2; US 6,740,118 B2; WO 01/01893 A1).

Bei Zwischenwirbelimplantaten mit in Einschubrichtung verlaufenden Finnen sind diese teilweise hoch ausgebildet, so daß eine gute Verankerung erfolgen kann, allerdings ist es dann notwendig, die Wirbelkörper entsprechend vorzubereiten, beispielsweise durch Einbringen einer Nut in den Wirbelkörper, in die die Finnen oder Rippen beim Einschieben des Zwischenwirbelimplantates eingeschoben werden können.

Insbesondere beim Einsetzen von Zwischenwirbelimplantaten auf gegenüberliegenden Seiten desselben Wirbelkörpers besteht dabei die Gefahr, daß durch die Nuten oder Einkerbungen der Wirbelkörper gespalten wird. Es ist daher häufig nicht möglich, auf beiden Seiten derartige Endplatten mit Vorsprüngen in Form von Finnen oder Rippen zu verwenden.

Andererseits bieten Endplatten, die nur Spikes oder niedrige Querrippen zur Verankerung aufweisen, nicht immer die gewünschte Lagestabilisierung, dies gilt insbesondere dann, wenn Wirbelkörper mit anomalen Formen angetroffen werden, die nicht vollflächig an den Endplatten anliegen, sondern nur partiell.

US-A-2004/0133278 offenbart ein Zwischenwirbelimplantat mit Endplatten, die unterschiedliche Verankerungsvorsprunge aufweisen.

Es ist Aufgabe der Erfindung, einen Bausatz vorzuschlagen, mit dem der Operateur auf die jeweils notwendigen Gegebenheiten für die Verankerung der Endplatten an benachbarten Wirbelkörpern Zugriff hat.

Diese Aufgabe wird bei einem Bausatz der eingangs beschriebenen Art erfindungsgemäß dadurch gelöst, daß er Endplatten eines ersten Typs umfaßt, die mindestens einen als Finne oder Rippe ausgebildeten Verankerungsvorsprung tragen, der parallel zur Einsetzrichtung des Zwischenwirbelimplantates verläuft, und Endplatten eines zweiten Typs, deren Verankerungsvorsprünge verschieden sind von Finnen oder Rippen, die parallel zur Einsetzrichtung verlaufen, und daß für jedes Zwischenwirbelimplantat sowohl Endplatten des ersten Typs als auch des zweiten Typs in dem Bausatz enthalten sind.

Der Operateur hat damit die Möglichkeit, noch während der Operation zu entscheiden, ob er das Implantat aus Endplatten des ersten Typs oder des zweiten Typs zusammensetzen will, gegebenenfalls können auch Endplatten des ersten und des zweiten Types gemeinsam an ein und demselben Zwischenwirbelimplantat Verwendung finden. So ist es beispielsweise möglich, beide Endplatten mit in Einschubrichtung verlaufenden Finnen oder Rippen auszustatten, beide Endplatten mit von diesen verschiedenen Verankerungsvorsprüngen auszustatten, es ist auch möglich ein Zwischenwirbelimplantat herzustellen, bei dem die obere Platte oder die untere Platte eine in Einsetzrichtung verlaufende Finne oder Rippe aufweist und die gegenüberliegende Platte verschieden ausgebildet ist.

Die Endplatten des zweiten Typs können zum Beispiel mindestens eine quer zur Einschubrichtung verlaufende Rippe tragen, wobei es vorteilhaft ist, wenn diese Rippe niedriger ist als die Rippe oder Finne des ersten Typs, die in Einsetzrichtung verläuft.

Bei einer anderen Ausgestaltung kann vorgesehen sein, daß die Endplatten des zweiten Typs mindestens einen Verankerungsstift tragen.

Es ist günstig, wenn der Bausatz für jede Größe einer Endplatte sowohl Endplatten des ersten Typs als auch Endplatten des zweiten Typs umfaßt, so daß der Operateur zusätzlich in dem Bausatz auch noch Endplatten unterschiedlicher Größe auswählen kann, die wahlweise dem ersten oder zweiten Typ angehören und die entweder in gleicher Größe oder aber auch in verschiedenen Größen miteinander kombiniert werden können.

Der Bausatz enthält also Endplatten unterschiedlicher Größe sowohl des ersten Typs als auch des zweiten Typs.

Die Erfindung betrifft auch ein Zwischenwirbelimplantat, welches eine obere und eine untere Endplatte zur Anlage an benachbarten Wirbelkörpern sowie zwischen den Endplatten angeordnete Lagerelemente zur verschwenkbaren gegenseitigen Abstützung der Endplatten aufweist, wobei die Endplatten an ihren den Wirbelkörpern zugewandten Außenseiten Verankerungsvorsprünge tragen.

Um ein solches Zwischenwirbelimplantat optimal an die anatomischen Gegebenheiten der benachbarten Wirbelkörper anpassen zu können, wird erfindungsgemäß vorgesehen, daß das Zwischenwirbelimplantat Endplatten eines ersten Typs umfaßt, die mindestens einen als Finne oder Rippe ausgebildeten Verankerungsvorsprung tragen, der parallel zur Einsetzrichtung des Zwischenwirbelimplantates verläuft, und Endplatten eines zweiten Typs, deren Verankerungsvorsprünge verschieden sind von Finnen oder Rippen, die parallel zur Einsetzrichtung verlaufen.

Die nachfolgende Beschreibung bevorzugter Ausführungsformen der Erfindung dient im Zusammenhang mit der Zeichnung der näheren Erläuterung. Es zeigen:
- Figur 1:: eine Seitenansicht eines zwischen zwei Wirbelkörpern eingesetz- ten Zwischenwirbelimplantats mit einer unteren Endplatte des er- sten Typs und einer oberen Endplatte des zweiten Typs;
- Figur 2:: das Zwischenwirbelimplantat der Figur 1 vor dem Zusammenbau und eine
- Figur 3:: perspektivische Ansicht eines Zwischenwirbelimplantates mit einer oberen Endplatte des ersten Typs und einer unteren Endplatte des zweiten Typs vor dem Zusammenbau.

Das in der Zeichnung dargestellte Zwischenwirbelimplantat 1 ist zum Einsatz in den Zwischenraum zwischen zwei benachbarten Wirbelkörpern 2, 3 bestimmt. Das Zwischenwirbelimplantat 1 weist eine obere Endplatte 4 auf und eine untere Endplatte 5, die außenseitig Anlageflächen zur Anlage an den benachbarten Wirbelkörpern 2, 3 aufweisen. Die beiden Endplatten 4 und 5 sind gegeneinander verschwenkbar aufeinander abgestützt, dazu ist zwischen die untere Endplatte 5 und die obere Endplatte 4 ein Lagerkörper 6 eingesetzt mit einer nach oben vorstehenden kugelkalottenförmigen Lagerfläche 7, die in eine komplementäre Lagerausnehmung 8 an der oberen Endplatte 4 eingreift.

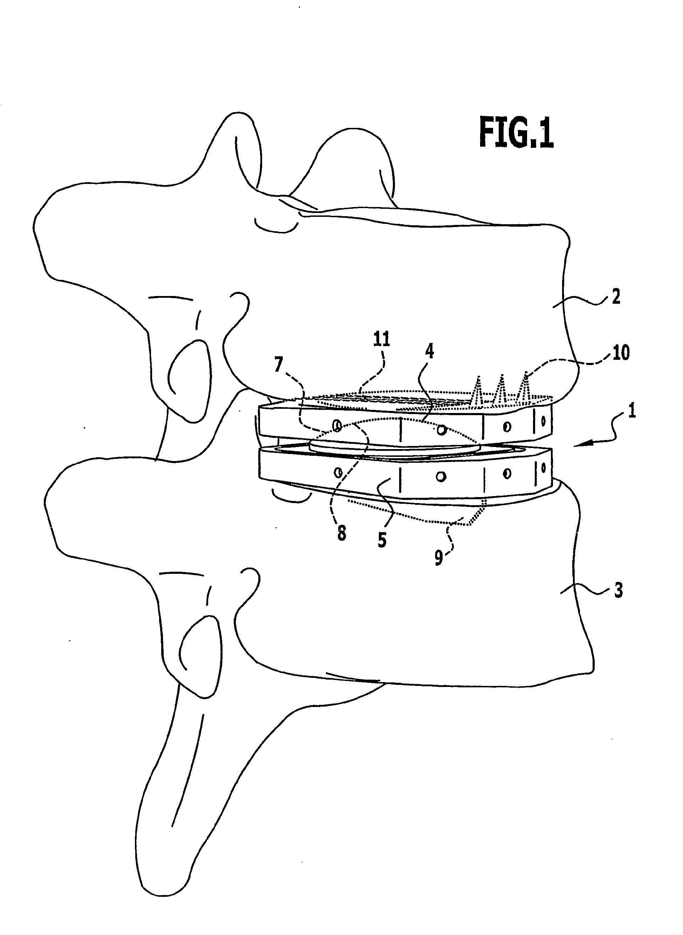

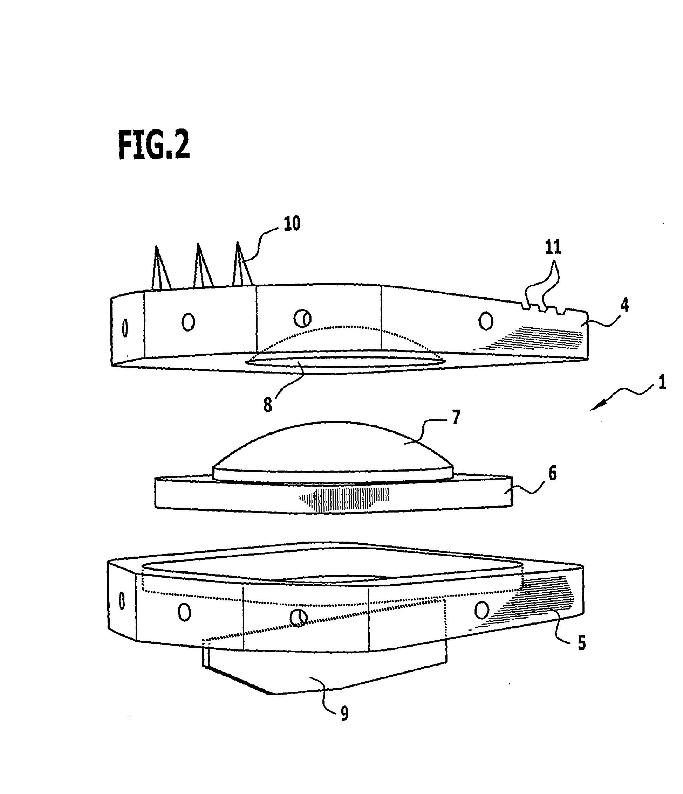

Bei dem Ausführungsbeispiel der Figuren 1 und 2 trägt die untere Endfläche an ihrer Außenseite eine mittige, senkrecht nach oben abstehende Finne oder Rippe 9, die sich im wesentlichen über die gesamte Endplatte 5 erstreckt und parallel zu einer Einsetzrichtung des Zwischenwirbelimplantates 1 verläuft. Diese Rippe 9 bildet einen Verankerungsvorsprung aus, der bei eingesetztem Zwischenwirbelimplantat 1 in den anliegenden Wirbelkörper 3 eindringt, der dazu entsprechend vorbereitet sein kann, z. B. durch Einbringen einer entsprechenden Nut.

Eine solche Endplatte mit einer Finne oder Rippe 9, die in Einsetzrichtung verläuft, wird nachstehend als Endplatte des ersten Typs bezeichnet.

Die gegenüberliegende obere Endplatte 4 weist bei dem in den Figuren 1 und 2 dargestellten Ausführungsbeispiel keine derartige Finne oder Rippe 9 auf, sondern statt dessen Verankerungsvorsprünge anderer Ausgestaltung, im dargestellten Ausführungsbeispiel in Form von nach oben abstehenden Spitzen 10 oder in Form von niedrigen Rillen oder Rippen 11, die quer zur Einsetzrichtung verlaufen. Diese Rippen entstehen durch Rillen, die in die Oberfläche der Endplatte eingearbeitet sind.

Eine solche Endplatte, die keinen Verankerungsvorsprung in Form einer in Einsetzrichtung verlaufenden Rippe oder Finne aufweist, wird nachstehend als Endplatte des zweiten Typs bezeichnet. Die Verankerungsvorsprünge können hier vielfältig gestaltet sein, wesentlich ist, daß es sich dabei nicht um in Einsetzrichtung verlaufende Rippen oder Finnen handelt, sondern um Verankerungsvorsprünge, die es auch ermöglichen, eine Endplatte des zweiten Typs in einer anderen Richtung in den Zwischenwirbelraum einzubringen als der normalen Einsetzrichtung, die üblicherweise in ventral-dorsaler Richtung verläuft.

Bei dem Ausführungsbeispiel der Figur 1 ist die untere Endplatte eine Endplatte des ersten Typs und die obere Endplatte eine Endplatte des zweiten Typs.

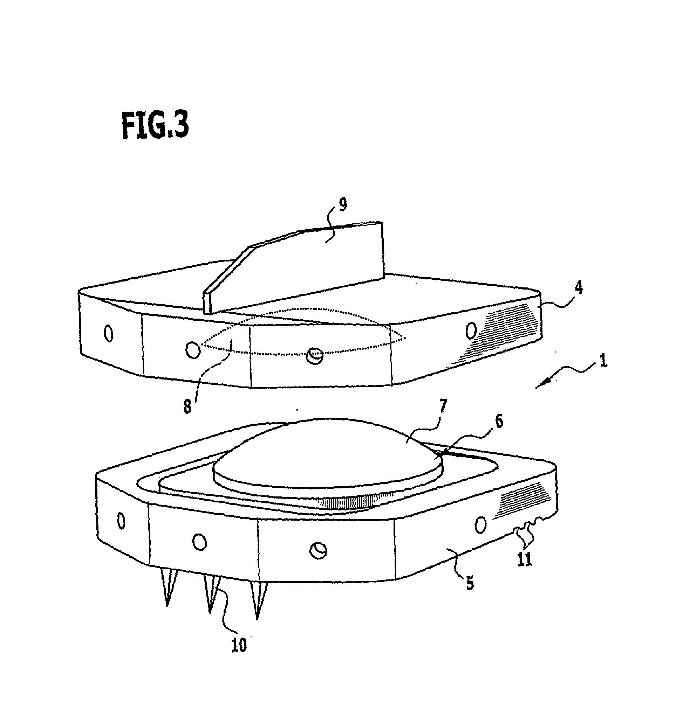

Bei dem Zwischenwirbelimplantat der Figur 3 ist die Anordnung umgekehrt gewählt, die obere Endplatte ist eine Endplatte des ersten Typs und die untere Endplatte eine Endplatte des zweiten Typs.

In einem erfindungsgemäßen Bausatz sind für jede Größe eines Zwischenwirbelimplantates sowohl obere Endplatten als auch untere Endplatten des ersten Typs und des zweiten Typs vorgesehen, so daß der Operateur diese Endplatten beliebig kombinieren kann. Er kann damit Zwischenwirbelimplantate herstellen, bei denen beide Endplatten Verankerungsvorsprünge des ersten Typs aufweisen oder beide Endplatten Verankerungsvorsprünge des zweiten Typs. Es ist auch möglich, Zwischenwirbelimplantate mit kombinierten Endplatten herzustellen, wie es anhand der Figuren 1 bis 3 erläutert worden ist.

Außerdem ist es günstig, wenn der Bausatz Endplatten unterschiedlicher Größen umfaßt, und zwar für jede Größe sowohl des ersten Typs als auch des zweiten Typs, so daß auch unterschiedlich große Zwischenwirbelimplantate mit Endplatten unterschiedlichen Typs hergestellt werden können, und zwar sowohl bei Zwischenwirbelimplantaten mit Endplatten der gleichen Größe als auch bei Zwischenwirbelimplantaten mit Endplatten unterschiedlicher Größe.

## Patentansprüche

1. Bausatz für ein Zwischenwirbelimplantat (1), welches eine obere und eine untere Endplatte (4, 5) zur Anlage an benachbarten Wirbelkörpern (2, 3) sowie zwischen den Endplatten (4, 5) angeordnete Lagerelemente zur verschwenkbaren gegenseitigen Abstützung der Endplatten (4, 5) aufweist, wobei die Endplatten (4, 5) an ihren den Wirbelkörpern (2, 3) zugewandten Außenseiten Verankerungsvorsprünge (10, 11) tragen,
wobei der Bausatz Endplatten (4, 5) eines ersten Typs umfaßt, die mindestens einen als Finne oder Rippe (9) ausgebildeten Verankerungsvorsprung tragen, der parallel zur Einsetzrichtung des Zwischenwirbelimplantates (1) verläuft, und Endplatten (4, 5) eines zweiten Typs umfaßt, **dadurch gekennzeichnet, daß** die Verankerungsvorsprünge (10, 11) der Endplatten des zweiten Typs verschieden sind von Finnen oder Rippen (9), die parallel zur Einsetzrichtung verlaufen, und daß für jedes Zwischenwirbelimplantat (1) sowohl Endplatten (4, 5) des ersten Typs als auch des zweiten Typs in dem Bausatz enthalten sind.

2. Bausatz nach Anspruch 1, **dadurch gekennzeichnet, daß** die Endplatten (4, 5) des zweiten Typs mindestens einen Verankerungsstift (10) tragen.

3. Bausatz nach Anspruch 1 oder 2, **dadurch gekennzeichnet, daß** die Endplatten (4, 5) des zweiten Typs mindestens eine quer zur Einsetzrichtung verlaufende Rippe oder Rinne (11) tragen.

4. Bausatz nach Anspruch 3, **dadurch gekennzeichnet, daß** die quer zur Einsetzrichtung verlaufenden Rinnen oder Rippen (11) des zweiten Typs niedriger sind als die Rippen oder Finnen (9) des ersten Typs.

5. Bausatz nach einem der voranstehenden Ansprüche, **dadurch gekennzeichnet, daß** er für jede Größe einer Endplatte (4, 5) sowohl Endplatten (4, 5) des ersten Typs als auch Endplatten (4,5) des zweiten Typs umfaßt.

6. Zwischenwirbelimplantat, welches eine obere und eine untere Endplatte zur Anlage an benachbarten Wirbelkörpern sowie zwischen den Endplatten angeordnete Lagerelemente zur verschwenkbaren gegenseitigen Abstützung der Endplatten aufweist, wobei die Endplatten an ihren den Wirbelkörpern zugewandten Außenseiten Verankerungsvorsprünge tragen, wobei das Zwischenswirbelimplantat eine Endplatte (4, 5) eines ersten Typs umfaßt, die mindestens einen als Finne oder Rippe (9) ausgebildeten Verankerungsvorsprung trägt, der parallel zur Einsetzrichtung des Zwischenwirbelimplantates (1) verläuft, und eine Endplatte eines zweiten Typs umfaßt, **dadurch gekennzeichnet, daß** die Verankerungsvorsprünge (10, 11) der Endplatte des zweiten Typs verschieden sind von Finnen oder Rippen (9), die parallel zur Einsetzrichtung verlaufen.

## Claims

1. Kit for an intervertebral implant (1) comprising an upper end plate (4) and a lower end plate (5) for placement on adjacent vertebral bodies (2, 3), and bearing elements arranged between the end plates (4, 5) for mutually pivotally supporting the end plates (4, 5), the end plates (4, 5) carrying anchoring projections (10, 11) on their outer sides which face the vertebral bodies (2, 3), the kit comprising end plates (4, 5) of a first type, which carry at least one anchoring projection configured as a fin or a rib (9) extending parallel to the direction of insertion of the intervertebral implant (1), and comprising end plates (4, 5) of a second type, **characterised in that** the anchoring projections (10, 11) of the end plates of the second type are different from fins or ribs (9) extending parallel to the direction of insertion, and **in that** the kit contains both end plates (4, 5) of the first type and end plates of the second type for each intervertebral implant (1).

2. Kit according to claim 1, **characterised in that** the end plates (4, 5) of the second type carry at least one anchoring pin (10).

3. Kit according to claim 1 or 2, **characterised in that** the end plates (4, 5) of the second type carry at least one rib or groove (11) extending transversely to the direction of insertion.

4. Kit according to claim 3, **characterised in that** the grooves or ribs (11) of the second type which extend transversely to the direction of insertion are lower than the ribs or fins (9) of the first type.

5. Kit according to one of the preceding claims, **characterised in that** the kit includes both end plates (4, 5) of the first type and end plates (4, 5) of the second type for each size of end plate (4, 5).

6. Intervertebral implant comprising an upper end plate and a lower end plate for placement on adjacent vertebral bodies, and bearing elements arranged between the end plates for mutually pivotally supporting the end plates, the end plates carrying anchoring projections on their outer sides which face the vertebral bodies, the intervertebral implant including an end plate (4, 5) of a first type, which carries at least one anchoring projection configured as a fin or a rib (9) extending parallel to the direction of insertion of the intervertebral implant (1), and comprising an end plate of a second type, **characterised in that** the anchoring projections (10, 11) of the end plate of the second type are different from fins or ribs (9) extending parallel to the direction of insertion.

## Revendications

1. Kit de pièces pour un implant intervertébral (1), qui comprend une plaque terminale supérieure et une plaque terminale inférieure (4, 5) pour venir en contact contre des corps vertébraux voisins (2, 3), ainsi que des éléments de montage agencés entre les plaques terminales (4, 5) pour soutenir les plaques terminales (4, 5) l'une par rapport à l'autre avec faculté de pivotement, dans lequel les plaques terminales (4, 5) portent, sur leurs faces extérieures tournées vers les corps vertébraux (2, 3), des saillies d'ancrage (10, 11),
dans lequel le kit de pièces comprend des plaques terminales (4, 5) d'un premier type, qui portent au moins une saillie d'ancrage réalisée sous forme d'ailette ou de nervure (9), qui s'étend parallèlement à la direction de mise en place de l'implant intervertébral (1), et comprend des plaques terminales (4, 5) d'un second type,
**caractérisé en ce que** les saillies d'ancrage (10, 11) des plaques terminales du second type sont différentes des ailettes ou des nervures (9), qui s'étendent parallèlement à la direction de mise en place, et **en ce que** pour chaque implant intervertébral (1), des plaques terminales (4, 5) du premier type tout comme des plaques terminales du second type sont contenues dans le kit de pièces.

2. Kit de pièces selon la revendication 1, **caractérisé en ce que** les plaques terminales (4, 5) du second type portent au moins une tige d'ancrage (10).

3. Kit de pièces selon la revendication 1 ou 2, **caractérisé en ce que** les plaques terminales (4, 5) du second type portent au moins une nervure ou une goulotte (11) s'étendant transversalement à la direction de mise en place.

4. Kit de pièces selon la revendication 3, **caractérisé en ce que** les rainures ou les nervures (11), s'étendant transversalement à la direction de mise en place, du second type, sont plus basses que les nervures ou les ailettes (9) du premier type.

5. Kit de pièces selon l'une des revendications précédentes, **caractérisé en ce qu'**il comprend, pour chaque taille de plaque terminale (4, 5), aussi bien des plaques terminales (4, 5) du premier type que des plaques terminales (4, 5) du second type.

6. Implant intervertébral, qui comprend une plaque terminale supérieure et une plaque terminale inférieure pour venir en contact contre des corps vertébraux voisins, ainsi que des éléments de montage agencés entre les plaques terminales pour soutenir les plaques terminales l'une par rapport à l'autre avec faculté de pivotement, dans lequel les plaques terminales portent des saillies d'ancrage sur leurs faces extérieures tournées vers les corps vertébraux, dans lequel l'implant intervertébral comprend une plaque terminale (4, 5) d'un premier type, qui porte au moins une saillie d'ancrage réalisée sous la forme d'une ailette ou d'une nervure (9), saillie qui s'étend parallèlement à la direction de mise en place de l'implant intervertébral (1), et comprend une plaque terminale d'un second type, **caractérisé en ce que** les saillies d'ancrage (10, 11) de la plaque terminale du second type sont différentes des ailettes ou des nervures (9) qui s'étendent parallèlement à la direction de mise en place.
